# EUROPÄISCHE PATENTANMELDUNG

(11) **EP 0 896 962 A1**
(43) Veröffentlichungstag der Anmeldung: **17.02.1999**
(21) Anmeldenummer: 98114502.2
(22) Anmeldetag: 01.08.1998
(51) Int. Cl.: C07D 277/34, C07D 277/36

(54) **Verfahren zur Herstellung von 2-substituierten 5-Formylthiazolen**

(30) Priorität: 14.08.1997 DE 19735197
(71) Anmelder: BAYER AG, 51368 Leverkusen (DE)
(72) Erfinder: Kraus, Helmut Dr., 51519 Odenthal (DE)

(57) **Zusammenfassung**

2-substituierte 5-Formylthiazole werden in vorteilhafter Weise hergestellt, indem man Halogenmalondialdehyde in Gegenwart eines Lösungsmittels und einer C₁-Verbindung der Formel (III) 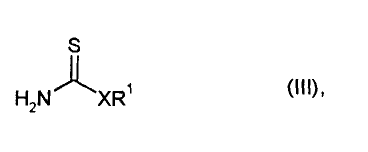 in der
- X: für Sauerstoff oder Schwefel und
- R¹: die in der Beschreibung angegebene Bedeutung haben,
umsetzt, wobei das Reaktionsgemisch weniger als 5 Gew.-% Wasser enthält.

## Beschreibung

Die vorliegende Erfindung betrifft ein verbessertes Verfahren zur Herstellung von 2-substituierten 5-Formylthiazolen.

2-substituierte 5-Formylthiazole sind wichtige Zwischenprodukte für die Herstellung von Pflanzenschutzmitteln (siehe z.B. EP-A-395 174).

Es ist bekannt, 4-substituierte 2-Alkoxy- bzw. 2-Thioalkyl-thiazole ausgehend von Halogenketonen und entsprechenden C₁-Bausteinen herzustellen (siehe Chem. Ber. 60, 2537 (1927)). Zur entsprechenden Herstellung von 5-substituierten Thiazolen wären jedoch statt der stabilen α-Halogenbetone die relativ instabilen α-Halogenaldehyde nötig. Normalerweise führt das zu Ausbeuteminderungen.

2-Amino-5-formylthiazol ist in geringer Ausbeute in wäßriger Lösung aus 2-Chlormalonaldehyd und Thioharnstoff zugänglich (siehe DE-A-1 182 234). Analog ist laut EP-A-125 094 auch 2-Thiomethyl-5-formylthiazol erhalten worden, jedoch fehlen eine Verfahrensverschreibung und Angaben zur Ausbeute.

Es wurde nun ein Verfahren zur Herstellung von 2-substituierten 5-Formylthiazolen der Formel 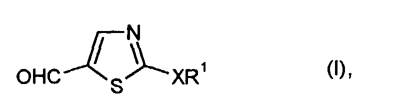 in der
- X: für Sauerstoff oder Schwefel und
- R¹: für C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl oder C₇-C₁₄-Aralkyl stehen,
gefunden,
das dadurch gekennzeichnet ist, daß man Halogenmalondialdehyde, die in einer tautomeren Form der Formel (II) entsprechen 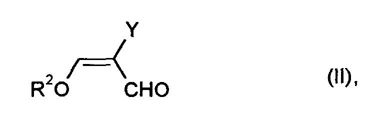 in der
- Y: für Fluor, Chlor, Brom oder Iod und
- R²: für Wasserstoff, ein Alkalimetall oder ein Äquivalent eines Erdalkalimetalls stehen,
in Gegenwart eines Lösungsmittels mit einer C₁-Verbindung der Formel (III) umsetzt 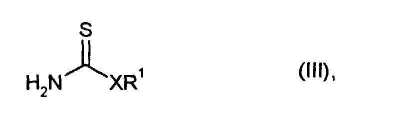 in der
- X und R¹: die bei Formel (I) angegebene Bedeutung haben,
wobei das Reaktionsgemisch weniger als 5 Gew.-% Wasser enthält.

In den Formeln (I) und (III) steht R¹ vorzugsweise für C₁-C₈-Alkyl. In Formel (II) stehen Y vorzugsweise für Chlor oder Brom und R² vorzugsweise für ein Alkalimetall, insbesondere für Natrium.

Verbindungen der Formel (II) sind auf bekannte Weise oder analog dazu, beispielsweise aus den entsprechenden Aminoethylenverbindungen herstellbar (siehe z.B. J. Org. Chem. 28, 3249 (1963) und Beispiel 4).

Verbindungen der Formel (III) mit X = Sauerstoff (= Xanthogenamide oder Thiocarbamidsäure-O-ester) sind auch auf bekannte Weise oder analog dazu, beispielsweise aus Ammoniumthiocyanat, einem Alkohol R¹OH und Schwefelsäure, herstellbar (siehe z.B. Chem. Ber. 115, 1252 (1982)).

Verbindungen der Formel (III) mit X = Schwefel (= Dithiocarbamidsäureester) sind ebenfalls auf bekannte Weise oder analog dazu, beispielsweise aus Ammoniumthiocarbamat und einem eine R¹-Gruppe enthaltenden Alkylierungsmittel, herstellbar (siehe z.B. Synthesis 1985, 948).

Bezogen auf 1 mol einer Verbindung der Formel (II) kann man beispielsweise 0,9 bis 1,25 mole einer Verbindung der Formel (III) einsetzen. Vorzugsweise beträgt diese Menge 1 bis 1,1 mol. Es wird darauf hingewiesen, daß hier und sonst in diesem Text der Hinweis auf eine Verbindung der Formel (II) auch die andere mögliche tautomere Form dieser Verbindung umfaßt.

Als Lösungsmittel für das erfindungsgemäße Verfahren kommen beispielsweise Ether wie Tetrahydrofuran, Diethylether und Methyl-tert.-butylether, Alkohole wie Ethanol und Isopropanol, Ester wie Essigsäureethyl- und -propylester, Nitrile wie Acetonitril, chlorierte Kohlenwasserstoffe wie Methylenchlorid, Di-, Tri- und Tetrachlorethan und Chlorbenzole und Carbonsäuren wie Ameisen-, Essig- und Propionsäure in Frage. Es können auch Lösungsmittelgemische eingesetzt werden. Bezogen auf 100 g einer Verbindung der Formel (II) kann man beispielsweise 50 bis 1000 g Lösungsmittel einsetzen.

Wenn man Verbindungen der Formel (II) mit R² = Alkali- oder Erdalkalimetall einsetzt, ist es bevorzugt als Lösungsmittel Carbonsäuren zu verwenden, insbesondere Ameisen- oder Essigsäure, beispielsweise 1 bis 5 mol Carbonsäure pro mol einer Verbindung der Formel (II).

Wenn man Carbonsäuren als Lösungsmittel einsetzt, ist es bevorzugt, diese durch Zugabe eines Puffersalzes abzupuffern. Bevorzugte Puffersalze sind Alkalisalze der jeweils eingesetzten Carbonsäure. Besonders bevorzugt ist der Einsatz von Ameisensäure/Natriumformiat und von Essigsäure/Natriumacetat. Pro mol Carbonsäure kann man z.B. 0,5 bis 5 mol Puffersalz einsetzen.

Das erfindungsgemäße Verfahren kann man z.B. bei Temperaturen im Bereich -20 bis +80°C durchführen. Bevorzugt sind Temperaturen von ±0 bis +65°C.

Es ist ein wesentliches Merkmal des erfindungsgemäßen Verfahrens, daß es in Gegenwart von weniger als 5, insbesondere weniger als 2 Gew.-% Wasser (bezogen auf das gesamte Reaktionsgemisch) durchgeführt wird. Es ist vorteilhaft, den Wassergehalt mit vertretbarem Aufwand weitgehend zu minimieren, z.B. auf einen Gehalt von weniger als 0,01 Gew.-% (bezogen auf das gesamte Reaktionsgemisch). Für das Entfernen letzter Wasseranteile ist häufig ein sehr hoher Aufwand erforderlich, der wirtschaftlich nicht mehr sinnvoll sein kann. Man setzt deshalb die benötigten Reagenzien und Hilfsstoffe im allgemeinen in getrockneter Form ein, jedoch nicht unbedingt in völlig wasserfreier Form. Die Verbindung der Formel (II) wird vor dem Einsatz durch Trocknen vorzugsweise weitgehend entwässert. Ebenso sollte die eingesetzte Verbindung der Formel (III) oder deren Lösung weitestgehend wasserfrei sein. Soweit Ameisensäure eingesetzt wird, kann diese maximal 4 Gew.-% Wasser enthalten. Vorzugsweise enthält sie maximal 0,3 bis 3 Gew.-% Wasser.

Das erfindungsgemäße Verfahren kann man beispielsweise so durchführen, daß man die Verbindung der Formel (II) in einem geeigneten Lösungsmittel vorlegt und die Verbindung der Formel (III) gelöst in einem geeigneten Lösungsmittel oder in geschmolzener Form zutropft.

Das nach der Durchführung des erfindungsgemäßen Verfahrens vorliegende Reaktionsgemisch kann man beispielseise aufarbeiten, indem man aus ihm die festen Bestandteile abtrennt, z.B. durch Filtration. Die dann vorliegende Rohlösung kann eingeengt, gegebenenfalls das Konzentrat neutralisiert und mit einem geeigneten Lösungsmittel, z.B. Dichlormethan, extrahiert werden. Nach der Klärung, der Konzentration des Extraktes und einer Kristallisation kann man das hergestellte 2-substituierte 5-Formylthiazol der Formel (I) in Reinheiten von 90 % und höher erhalten.

Eine besonders bevorzugte Ausführungsform der vorliegenden Erfindung besteht darin, daß man Natriumchlormalondialdehyd in Ameisensäure/Natriumformiat oder Essigsäure/Natriumacetat mit gegebenenfalls gelöstem Xanthogenamid bzw. 5-Alkyldithiocarbonat in Gegenwart von 0,5 bis 2 Gew.-% Wasser (bezogen auf das gesamte Reaktionsgemisch) umsetzt. Man erhält dann im allgemeinen Reaktionsausbeuten an 2-substituierten 5-Formylthiazolen der Formel (I) im Bereich 65 bis 90 % der Theorie. Die isolierten Ausbeuten können dann z.B. im Bereich 55 bis 80 % der Theorie liegen.

Das erfindungsgemäße Verfahren liefert 2-substituierte 5-Formylthiazole auf einfache, nacharbeitbare Weise in guten Ausbeuten.

### Beispiele

### Beispiel 1

64,3 g Natriumchlormalondialdehyd (erhalten gemäß Beispiel 4) und 34 g Natriumformiat wurden in 300 ml Ameisensäure vorgelegt und bei 20°C eine Lösung von 50,5 g Ethylxanthogenamid in 300 ml Essigester zugetropft. Das Reaktionsgemisch enthielt 1,5 Gew.-% Wasser. Nach dem Rühren über Nacht wurde am Rotationsverdampfer bei 20 mbar und 50°C Badtemperatur eingeengt, mit 200 ml Wasser versetzt und mit wäßriger Natronlauge ein pH-Wert von 5 eingestellt. Anschließend wurde mit Methylenchlorid extrahiert und mit Celite® und Tonsil® geklärt. Nach dem Einengen resultierten 66,9 g eines Öls, das langsam durchkristallisierte. Es handelte sich dabei um 2-Ethoxy-5-formylthiazol mit einem Schmelzpunkt (MTBE) von 66,5°C.
- ¹H-NMR (CDCl₃):: 1,48 (t, 3H); 4,57 (q, 2H); 7,84 (s, 1H); 9,82 (s, 1H) ppm
- ¹³C-NMR:: 182,4; 180,8; 149,4; 133,0; 69,5; 14,7 ppm

### Beispiel 2

Analog Beispiel 1 wurden 0,5 mol Natriumchlormalondialdehyd mit 0,5 mol S-Methyldithiocarbamat bei einem Wassergehalt des Reaktionsgemisches von 1,8 % umgesetzt. Man erhielt in einer Ausbeute von 88 % der Theorie 2-Thiomethyl-5-formylthiazol mit einem Schmelzpunkt von 82°C.
- ¹H-NMR (CDCl₃):: 2,75 (s, 3H); 8,21 (s, 1H); 9,90 (s, 1H) ppm
- ¹³C-NMR:: 181,8; 178,7; 152,6; 139,5; 17,6 ppm

### Beispiel 3

3,44 g Natriumbrommalondialdehyd und 1,36 g Natriumformiat wurden in 30 ml Ameisensäure vorgelegt und bei 10°C 9 g aufgeschmolzenes Ethylxanthogenamid zugetropft. Das Reaktionsgemisch enthielt 1,2 % Wasser. Nach 20 Stunden Rühren bei Raumtemperatur wurde analog Beispiel 1 aufgearbeitet und 2-Ethoxy-5-formylthiazol in einer Ausbeute von 73 % der Theorie erhalten.

### Beispiel 4

### Herstellung des Ausgangsprodukts Natriumchlormalondialdehyd (nicht erfindungsgemäß)

134 g 3-Dimethylaminochloracrolein wurde mit einem Äquivalent Natronlauge 1 Stunde lang auf 70°C erhitzt. Nach dem Abkühlen wurde der Niederschlag abfiltriert und mit Ethanol gewaschen. So wurde Natriumchlormalondialdehyd-Trihydrat erhalten. Dieses wurde in einem Trockenschrank bei 100°C und 300 mbar 1 Stunde lang getrocknet.
- ¹H-NMR (d-DMSO):: 8,6 ppm (s).

## Patentansprüche

1. Verfahren zur Herstellung von 2-substituierten 5-Formylthiazolen der Formel (I) 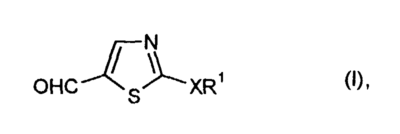 in der
X für Sauerstoff oder Schwefel und
R¹ für C₁-C₈-Alkyl, C₃-C₈-Alkenyl, C₂-C₈-Alkoxyalkyl, C₄-C₈-Alkoxyalkenyl, C₃-C₈-Cycloalkyl, C₆-C₁₂-Aryl oder C₇-C₁₄-Aralkyl stehen,
dadurch gekennzeichnet, daß man Halogenmalondialdehyde, die in einer tautomeren Form der Formel (II) entsprechen 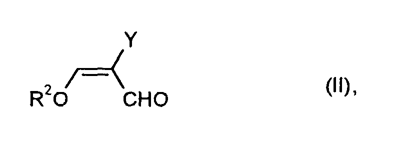 in der
Y für Fluor, Chlor, Brom oder Iod und
R² für Wasserstoff, ein Alkalimetall oder ein Äquivalent eines Erdalkalimetalls stehen,
in Gegenwart eines Lösungsmittels mit einer C₁-Verbindung der Formel (III) umsetzt 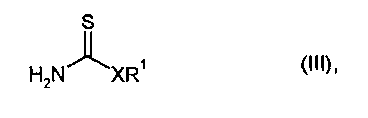 in der
X und R¹ die bei Formel (I) angegebene Bedeutung haben,
wobei das Reaktionsgemisch weniger als 5 Gew.-% Wasser enthält.

2. Verfahren nach Anspruch 1, dadurch gekennzeichnet, daß das Reaktionsgemisch weniger als 2 Gew.-% Wasser enthält.

3. Verfahren nach Ansprüchen 1 und 2, dadurch gekennzeichnet, daß in den Formeln (I) und (III) R¹ für C₁-C₈-Alkyl und in der Formel (II) Y für Chlor oder Brom und R² für ein Alkalimetall stehen.

4. Verfahren nach Ansprüchen 1 bis 3, dadurch gekennzeichnet, daß man, bezogen auf 1 mol einer Verbindung der Formel (II) 0,9 bis 1,25 mole einer Verbindung der Formel (III) einsetzt.

5. Verfahren nach Ansprüchen 1 bis 4, dadurch gekennzeichnet, daß man als Lösungsmittel Ether, Alkohole, Ester, Nitrile, chlorierte Kohlenwasserstoffe, Carbonsäuren und/oder Amine einsetzt.

6. Verfahren nach Ansprüchen 1 bis 5, dadurch gekennzeichnet, daß man beim Einsatz von Verbindungen der Formel (II) mit R² = Alkali- oder Erdalkalimetall als Lösungsmittel Carbonsäuren verwendet.

7. Verfahren nach Anspruch 6, dadurch gekennzeichnet, daß man Puffersalze zusetzt.

8. Verfahren nach Ansprüchen 1 bis 7, dadurch gekennzeichnet, daß man es bei Temperaturen im Bereich -20 bis +80°C durchführt.
